# EUROPÄISCHE PATENTANMELDUNG

(11) **EP 1 334 736 A1**
(43) Veröffentlichungstag der Anmeldung: **13.08.2003**
(21) Anmeldenummer: 03001486.4
(22) Anmeldetag: 23.01.2003
(51) Int. Cl.: A61L 9/012, A61L 9/014, A61L 9/04, B01D 53/02, A61L 9/16

(54) **Verfahren zum Desodorieren von Tierzuchtanlagen**

(30) Priorität: 29.01.2002 DE 10203339; 14.03.2002 DE 10211165
(71) Anmelder: Air & D-Sarl, 67190 Rosheim (FR)
(72) Erfinder: Wuest, Robert, 67190 Rosheim (FR)
(74) Vertreter: Grussdorf, Jürgen, Dr.

(57) **Zusammenfassung**

Die Erfindung betrifft ein Verfahren und eine Vorrichtung zum Desodorieren von Tierzuchtanlagen durch Behandeln der verunreinigten Luft mit aktiven Agentien, die mit den in der Luft enthaltenen übelriechenden Substanzen reagieren oder diese maskieren.
Die aktiven Agentien sind in einer Matrix aus einem vernetzten, hydrophile Gruppen enthaltenden Polymeren verteilt und bilden mit diesem zusammen eine schwammartige Masse, aus der die aktiven Agentien langsam freigesetzt werden und verdunsten.

## Beschreibung

Die Erfindung betrifft ein Verfahren zum Desodorieren von Tierzuchtanlagen durch Behandeln der verunreinigten Luft mit aktiven Agentien, die mit den in der Luft enthaltenen übelriechenden Substanzen reagieren bzw. diese maskieren.

Aus wirtschaftlichen Gründen und bedingt durch die Anforderungen des Marktes ist die Massentierhaltung unvermeidlich geworden. Dies hat zur Folge, dass die Ställe immer größer werden. Insbesondere bei der Schweinezucht führt dies zu starker Bildung von üblen Gerüchen, was nicht nur in der Schweinemastanlage selbst sondern auch in der Umgebung zu erheblichen Belästigungen führt. Es besteht daher das Bedürfnis nach einer effizienten und schnell wirkenden Beseitigung oder zumindest starker Verminderung dieser üblen Gerüche.

Die daraus resultierende Aufgabe wird durch das erfindungsgemäße Verfahren gelöst. Gegenstand der Erfindung ist demzufolge ein Verfahren zum Desodorieren von Tierzuchtanlagen durch Behandeln der verunreinigten Luft mit aktiven Agentien, die mit den in der Luft enthaltenen übelriechenden Substanzen reagieren oder diese maskieren, wobei die aktiven Agentien in einer Matrix aus einem vernetzten, hydrophile Gruppen enthaltenden Polymeren verteilt sind und mit diesem zusammen eine schwammartige Masse bilden, aus der die aktiven Agentien langsam freigesetzt werden und verdunsten.

Tierzuchtanlagen im Sinne der Erfindung umfassen nicht nur die Ställe selbst sondern auch in der Anlage befindliche Güllebehälter. Besonders vorteilhaft wird die Erfindung zum Desodorieren von Schweineställen und den zugehörigen Güllebehältern angewandt; sie ist natürlich auch in anderen Tierzuchtanlagen, z. B. für Kühe, Geflügel und Kaninchen anwendbar.

Geeignete Matrix-Polymere sind vernetzte (Meth-)Acrylat-Polymere und vorzugsweise vernetzte maleinisierte oder epoxidierte Polymere.

Bevorzugt sind Kondensationsprodukte aus einem maleinisierten oder epoxidierten Polymeren und einem Vernetzer, vorzugsweise einem Polyamin. Geeignete Polymere sind z. B. Umsetzungsprodukte aus einem Polydien, z. B. Polybutadien, Polyoctadien und Soyabohnenöl, mit Maleinsäureanhydrid, ferner Copolymere von Olefinen, wie Ethylen, mit Maleinsäureanhydrid, sowie epoxidiertes Polybutadien. Bevorzugte Vernetzer sind Polyamine, insbesondere Polyoxypropylendiamin und Polyoxypropylentriamin. Daneben sind auch Harnstoff, Polyethylenimin, sowie Triethylenglykol als Vernetzer geeignet. Die Vernetzungsreaktion erfolgt vorzugsweise in alkoholischer Lösung, z. B. in Dipropylenglykol, bei erhöhter Temperatur. Als hydrophile Gruppen wirken insbesondere die von den Polyoxyalkylenpolyaminen stammenden -CRH-O- Gruppen, daneben auch die Maleinsäureanhydrid- und Carboxylgruppen oder Epoxidgruppen bzw. die -NR-CO- Gruppen des vernetzten Polymeren.

Eine andere Klasse von vernetzten Polymeren sind Copolymerisate von monofunktionellen (Meth-)Acrylat-Monomeren, z. B. Hydroxyethylacrylat oder Poly(propylenoxid)(ethylenoxid)monomethacrylat, mit einem polyfunktionellen (Meth-)Acrylat-Monomeren, z. B. Ethylenglykoldimethacrylat oder Polyethylenglykol-400- dimethacrylat. Die Herstellung der vernetzten (Meth-)Acrylat-Polymeren erfolgt durch radikalische Copolymerisation der Monomeren.

In beiden Fällen sind die vernetzten Polymeren in der Lage, Flüssigkeiten und Gase, z. B. die aktiven Agentien zu adsorbieren, wobei sich eine offenzellige Schwammstruktur ausbildet. Das vernetzte Polymere weist ein räumliches Netzwerk mit Poren auf, in dem die flüchtigen Fremdsubstanzen aufgesaugt und adsorbiert werden können, so dass das Polymere wie ein Schwamm anquillt. Im angequollenen Zustand besteht das dreidimensionale Netzwerk aus Elementarzellen, die im Mittel ein Volumen von 1 bis 1000 nm³, vorzugsweise von 3 bis 200 nm³, aufweisen.

Das vernetzte Polymere ist erfindungsgemäß mit einem aktiven Agens beladen und bildet mit diesem eine schwammartige Masse. Das aktive Agens wird daraus langsam freigesetzt und kann dann mit in der Anlage vorhandenen übelriechenden Substanzen, z. B. Aminen, Ammoniak und Schwefelverbindungen reagieren, diese reduzieren bzw. maskieren. Die aktiven Agentien sind meist flüssige Aldehyde, Ketone, Alkohole oder Ester, beispielsweise Vanillin, Eugenol, Thymol, Geraniol, Kampferöl, Citronellol, Linanol, Menthol, Cumarin, Citral, Alpha-Pinen, Nerylacetat, Linalylacetat, Butylhydroxytoluol und Salicylsäurebenzylester, C₇- bis C₁₂-Aldehyde, ferner natürliche ölige Essenzen, wie Harzöl, sowie Mischungen. Neben eigentlichen chemischen Reaktionen, z. B. zwischen Ammoniak und Aldehyden, kann es auch zu Bindungen durch elektrostatische oder van der Waals'sche Kräfte kommen, wodurch die Geruchwahrnehmbarkeit zumindest herabgesetzt wird.

Bevorzugt werden die aktiven Agentien bei der Herstellung der vernetzten Polymeren durch Kondensation bzw. Polymerisation zugesetzt, man kann aber auch das vernetzte Polymere mit den aktiven Agentien tränken und damit anquellen. Das aktive Agens sollte in der schwammartigen Masse in Mengen von 10 bis 90 Gew.-%, vorzugsweise von 40 bis 80 Gew.-% enthalten sein.

Die schwammartige Masse kann neben der Polymermatrix und den aktiven Agentien noch weiter Zusatzstoffe, z. B. 1 bis 20 Gew.-% Flammschutzmittel, wie Zucker, Bromverbindungen oder Azodicarbonamid, 1 bis 8 Gew.-% Wasser, sowie Pulver zur Verminderung des Verbackens und Sublimationshilfsmittel enthalten.

Wesentlich ist, dass das aktive Agens mit den zur Anwendung kommenden vernetzten Polymeren so abgestimmt ist, dass es nur sehr langsam aus der schwammartigen Masse freigesetzt wird und seine Wirkung mindestens drei Tage, vorzugsweise mindestens eine Woche und insbesondere mehr als einen Monat lang beibehält. Das freigesetzte aktive Agens kann mit den übelriechenden Substanzen in der Gasphase reagieren und/oder den üblen Geruch überdecken. Darüberhinaus ist die schwammartige Masse in der Lage, gasförmige übelriechende Substanzen zu absorbieren und dadurch aus der Luft zu entfernen.

Die schwammartige Masse, welche die aktiven Agentien enthält, kann in Form von Kugeln, Spänen oder Granulat zur Anwendung kommen. Sie wird jedoch bevorzugt in Form von Krümeln, Streifen oder Platten mit einer Dicke von 0,2 bis 5 cm, insbesondere von 0,5 bis 3 cm, eingesetzt. Die Platten oder Streifen können als solche im Stall oder in den Güllebehältern aufgehängt werden, bevorzugt werden sie aber auf Gitter oder Netzen aufgelegt. Besonders günstig ist es, dabei mehrere Gitter übereinander oder nebeneinander auf einem Gestell anzuordnen und dieses in einen Ventilationskasten einzubauen, der im Stall oder am Lufteingang zum Stall angebracht ist. In letzterem bewirkt die angesaugte Frischluft die Verdunstung der aktiven Agentien. Es ist auch möglich, Platten oder Steifen auf einem Netz zwischen zwei parallele Platten einzulegen, durch die die Luft, zweckmäßigerweise durch ein Gebläse beschleunigt, streichen kann. Wenn sich Tierzuchtanlagen in der Nähe von Wohnbebauung befinden, kann die ausgeblasene Abluft aus den Ställen zu erheblichen Belästigungen führen. Diese können vermindert werden, wenn die erfindungsgemäße schwammartige Masse in den Abluftkamin eingelagert wird. In Güllebehältern kann man Blöcke, Platten oder Streifen auch auf Schaumstoffplatten auflegen, die auf der Gülle schwimmen.

### Beispiel 1

21 g maleinisiertes Polybutadien (Umsetzungsprodukt von flüssigem Polybutadien mit Maleinsäureanhydrid - LITHENE von Revertex) wurden mit 79 g einer Mischung von öligen Essenzen als aktives Agens bei 45° C vermischt (Mischung A). 94 g des aktiven Agens und 7,5 g Polyoxypropylentriamin (MG 400) wurden vermischt (Mischung B). Die Mischungen A und B wurden zusammengerührt.
Die erhaltene schwammartige Masse wurde in Platten von 1 cm Dicke, 15 cm Breite und 20 cm Länge geschnitten und auf Metallgitter aufgelegt. Mehrere Gitter wurden übereinander auf einem Gestell angeordnet. Dieses Gestell wurde vor dem Lufteingangsschacht eines Schweinestalls angebracht, wo es über mehr als einen Monat hinweg seine desodorierende Wirkung entfaltete.

### Beispiel 2

Die schwammartige Masse nach Beispiel 1 wurde in Platten von 2 cm Dicke, 20 cm Breite und 20 cm Länge geschnitten und auf ein Metallnetz aufgelegt. Dieses Netz wurde zwischen zwei parallele Kunststoffplatten mit einem Abstand von 10 cm eingebracht. Zehn solcher Platten wurden an der Decke eines Güllebehälters aufgehängt.

## Patentansprüche

1. Verfahren zum Desodorieren von Tierzuchtanlagen durch Behandeln der verunreinigten Luft mit aktiven Agentien, welche mit den in der Luft enthaltenen übelriechenden Substanzen reagieren und/oder diese maskieren, **dadurch gekennzeichnet, dass** die aktiven Agentien in einer Matrix aus einem vernetzten, hydrophile Gruppen enthaltenden Polymeren verteilt sind und mit diesem zusammen eine schwammartige Masse bilden, aus der die aktiven Agentien langsam freigesetzt werden und verdunsten.

2. Verfahren nach Anspruch 1, **dadurch gekennzeichnet, dass** das vernetzte Polymere ein Kondensationsprodukt aus einen maleinisierten oder epoxidierten Polymeren und einem Vernetzer, vorzugsweise einem Polyamin, ist.

3. Verfahren nach Anspruch 1, **dadurch gekennzeichnet, dass** das vernetzte Polymere ein Copolymerisat aus einem monofunktionellen (Meth-)Acrylat-Monomeren und einem polyfunktionellen (Meth-)Acrylat-Monomeren ist.

4. Verfahren nach Anspruch 1, **dadurch gekennzeichnet, dass** die aktiven Agentien über einen Zeitraum von mindestens drei Tagen hinweg aus der schwammartigen Masse freigesetzt werden.

5. Verfahren nach Anspruch 1, **dadurch gekennzeichnet, dass** die aktiven Agentien in der schwammartigen Masse in einer Menge von 10 bis 90 Gew.-% enthalten sind.

6. Verfahren nach Anspruch 1, **dadurch gekennzeichnet, dass** die aktiven Agentien Aldehyde, Ketone, Alkohole, Ester, Terpene oder natürliche ölige Essenzen sind.

7. Verfahren nach Anspruch 5, **dadurch gekennzeichnet, dass** die schwammartige Masse zusätzlich Flammschutzmittel, Sublimationshilfsmittel und Wasser enthält.

8. Verfahren nach Anspruch 1, **dadurch gekennzeichnet, dass** die schwammartige Masse, welche die aktiven Agentien enthält, in Form von Krümeln, Streifen oder Platten mit einer Dicke von 0,2 bis 5 cm eingesetzt wird.

9. Verfahren nach Anspruch 8, **dadurch gekennzeichnet, dass** die Krümel, Streifen oder Platten auf Netze oder Gitter aufgelegt sind.

10. Verfahren nach Anspruch 9, **dadurch gekennzeichnet, dass** mehrere Gitter übereinander oder nebeneinander auf einem Gestell angeordnet sind.

11. Verfahren nach Anspruch 9, **dadurch gekennzeichnet, dass** die Netze oder Gitter, die gegebenenfalls in einem Gestell angeordnet sind, in einem Stall oder in dessen Luftein- oder ausgängen aufgehängt sind.

12. Verfahren nach Anspruch 8, **dadurch gekennzeichnet, dass** die Netze oder Gitter zwischen zwei Platten eingelagert sind.

13. Verfahren nach Anspruch 1 zum Desodorieren von Schweinemastanlagen.

14. Vorrichtung zur Durchführung des Verfahrens nach Anspruch 1, umfassend ein Gestell, auf dem mehrere Gitter übereinander oder nebeneinander angeordnet sind, auf denen Krümel, Platten oder Streifen aus einer schwammartigen Masse aufgelegt sind, welche desodorierende Agentien enthält.
